# EUROPEAN PATENT APPLICATION

(11) **EP 4 035 699 A1**
(43) Date of publication of application: **03.08.2022**
(21) Application number: 21153864.0
(22) Date of filing: 27.01.2021
(51) Int. Cl.: A61L 27/36, A61L 27/50, A61L 27/54

(54) **PROCESS FOR PRODUCING ACELLULARIZED IMPLANTS**

(71) Applicant: Medizinische Hochschule Hannover, 30625 Hannover (DE)
(72) Inventor: Hilfiker, Andres, 30625 Hannover (DE); Büttner, Falk, 30625 Hannover (DE); Smart, Isabel, 30625 Hannover (DE); Haverich, Axel, 30625 Hannover (DE)
(74) Representative: Taruttis, Stefan Georg

(57) **Abstract**

The invention provides a process for producing acellular implants from a matrix material of non-human animal origin by decellularizing a matrix material to generate an acellular matrix, oxidizing the acellular matrix by treatment of the acellular matrix with an oxidant, to generate an oxidized acellular matrix, and reacting the oxidized acellular matrix with an agent that is reactive with carbonyl groups and is non-antigenic.

## Description

The present invention relates to an in vitro process for generating an acellular implant from a decellularized matrix of non-human animal origin, and to the acellular implant obtainable by the process. The decellularized matrix is e.g. of porcine origin. The decellularized matrix of non-human animal origin is e.g. a decellularized heart valve, decellularized blood vessel, decellularized pericardium, decellularized cartilage, decellularized skin, decellularized cornea or other.

The process has the advantage of producing an implant that has sufficient stability as a structural implant; the implant has a structure that allows for colonization and growth by cells of the recipient, preferably also allowing for re-structuring and de-novo synthesis of its extracellular matrix constituents, e.g. collagen constituents, by cells of the recipient. The implant obtained by the process has the advantage of a significantly reduced antigenicity in a human recipient, resulting in a minimized or absent immune reaction by the recipient against the implant. The minimized or absent antigenicity of the implant is caused by a significantly reduced concentration of exposed antigenic molecules, e.g. its surface-exposed molecules essentially consist of non-immunogenic molecules. The recipient is a human patient in need of an implant, preferably a non-mature human patient who is still growing.

### State of the art

Carpentier et al., Valvular heterografts, 467-483 (1969) for reducing adverse immunological reactions of the recipient against heterografts, e.g. heart valves of animal origin, describes elimination of soluble proteins from an animal heart valve by washing or electrodialysis, followed by denaturing mucopolysaccharides and structural glycoproteins by oxidation using sodium periodate, which creates free aldehydes that cross-link with free amino groups of other molecules. This oxidation is stopped after 24h with ethylene glycol. Then the heart valve is placed in glutaraldehyde buffered solution for cross-linking other free amino groups, e.g. of lysine. The heart valve can be stored in the glutaraldehyde solution at 4°C for several months. The cross-linkage significantly reduces antigenicity.

Cross-linked matrix materials have the advantage of providing a combination of reduced antigenicity and a good mechanical stiffness, which is e.g. desirable in mechanically loaded implants like heart valves.

It was found that cross-linked implants have the disadvantage of not growing within the recipient, possibly due to the cross-linkages preventing ingrowth of cells of the recipient.

Simon et al., European Journal of Cardio-Thoracic Surgery 1002-1006 (2003) report on structural failure of SYNERGRAFT porcine heart valves in implant recipients, resulting in death of 1 of 4 child patients after 7 days, and 1 of 4 after 6 weeks, and 1 of 4 after 1 year after implantation, the 4^{th} heart valve implant was explanted shortly afterwards. These porcine heart valves were decellularized and showed strong inflammatory responses.

EP 1 881 854 A1 describes decellularization of heart tissue by treatment with aqueous Na-deoxycholate followed by osmotic treatment with distilled water and subsequently with physiological salt solution.

### Object of the invention

The object of the invention is to provide an alternative process for producing implants from decellularized matrix of non-human animal origin to provide acellular implants that have sufficient mechanical strength, that allow the colonization by cells of the recipient, and that have a low or absent antigenicity in a human recipient.

### Description of the invention

The invention achieves the object by the features of the claims, and especially provides a process for producing acellular implants from a matrix material of non-human animal origin by decellularizing a matrix material to generate an acellular matrix, oxidizing the acellular matrix by treatment of the acellular matrix with an oxidant, which preferably is sodium periodate, in aqueous solution, to generate an oxidized acellular matrix, and reacting the oxidized acellular matrix with an agent that is reactive with carbonyl groups and is non-antigenic. Accordingly, the agent is also referred to as saturating agent. Preferably, the saturating agent is a compound with a carbonyl-reactive group, e.g. a primary amine, an aminooxy-group or a hydrazide group, preferably selected from aminooxy-biotin, biotin hydrazide, glycine or any other amino acid or a peptide or protein, e.g. optionally having immunomodulatory properties. For the saturating agent being a primary amino compound, the step of reacting the oxidized acellular matrix with the saturating agent can be in the presence of a catalyst, e.g. aniline or anthranilic acid.

Generally, the process for producing an acellular implant from a matrix material of non-human animal origin can comprise or consist of the steps of decellularizing a matrix material, e.g. a freshly isolated or cryopreserved tissue containing extracellular matrix, e.g. a collagen structure, to generate an acellular matrix, a step of oxidizing the acellular matrix by treatment of the acellular matrix with an oxidant, optionally in the presence of a further catalyst, to generate an oxidized acellular matrix, optionally a step to remove excess of oxidant by another agent such as ethylene glycol, and a step of reacting the oxidized acellular matrix with an agent that is reactive with carbonyl groups and that preferably is non-antigenic in humans, optionally in the presence of a further catalyst. A preferred agent is a molecule also found in humans, e.g. biotin, glycine or other amino acids, a peptide or a protein. These other amino acids can e.g. be lysine having a carbonyl-reactive amino group on its side chain, a peptide or a protein can e.g. be a peptide or a protein with immunomodulatory function, e.g. human leukozyte antigen or human inhibitory ligand PD-L1 (CD 274) or a peptide or protein of the extracellular matrix, e.g. collagen or laminin.

Preferably, the oxidant has activity to oxidize glycans but not to oxidize proteins.

The step of oxidizing the acellular matrix is preferably adapted to oxidize only glycans of the acellular matrix, e.g. without oxidizing other components of the matrix like e.g. peptide bonds, side chains of amino acids or fatty acids. For oxidizing only glycans of the acellular matrix, the oxidizing agent preferably is sodium periodate.

Optionally, in the step of oxidizing the acellular matrix the saturating agent may be present in order to further minimize cross-linking of the matrix even prior to the step of reacting the oxidized acellular matrix with a saturating agent.

The process of oxidation and saturation has been found to efficiently minimize the detection of the acellular matrix by glycan specific lectins or antibodies as well as by preformed antibodies present in human serum. Currently, it is assumed that the saturating agent binds to reactive groups, especially to carbonyl groups which were specifically generated by the activity of the oxidant acting on glycans, and covers the former carbonyl-containing molecules, e.g. the remaining portions of glycans, preventing them to act as antigenic sites. This process can also mask new antigens, e.g. new antigens that were generated by the oxidation.

Preferably, the step of reacting the oxidized acellular matrix with the saturating agent is performed immediately after removing the oxidant from the oxidized acellular matrix in order to minimize cross-linking reactions, e.g. between carbonyl groups formed by the oxidation and carbonyl-reactive groups of the matrix, e.g. amino groups or hydroxyl groups.

Oxidizing the acellular matrix and reacting it with the saturating agent has been found to result in a masking of antigenic sites, which can include complete or partial glycans that are at least partially oxidized.

It is an advantage of the process according to the invention that it generates no or significantly reduced cross-linkages within the implant. The avoidance of cross-linking the matrix is of advantage, because cross-linking has been found to prevent the infiltration of the implant by cells of the recipient, and to therefore impair the remodeling of the extracellular matrix and growth of the implant within the recipient. Additionally, in preliminary analyses, it has been found that tissue that has been crosslinked by glutaraldehyde fixation shows altered mechanical properties. This enhanced rigidity is likely to interfere with physiological hemodynamics.

The cross-linkages that can be generated by a process of oxidizing an acellular matrix without the subsequent step of reacting it with a saturating agent that binds to carbonyl groups e.g. comprise artificial cross-linkages such as imide bonds between carbonyl groups generated on the glycans and amino groups of the proteinaceous compounds of the matrix.

The acellular matrix is preferably generated by decellularizing the matrix material of non-human animal origin, preferably by treatment of an original non-human animal matrix using enzymatic and/or detergent treatment, e.g. trypsin, TritonX100, sodium dodecyl sulphate (SDS), and/or sodium deoxycholate (SD) to remove cells.

An acellular matrix, herein also referred to as an implant, which is produced by the process of the invention has the advantage that its glycans are not detected by pre-formed human antibodies. The inaccessibility of the glycans of the acellular matrix of the invention towards pre-formed antibodies can e.g. be determined by the acellular matrix being non-reactive to human sera or α-Gal specific lectin or α-Gal specific antibody, e.g. by Isolectin B4 and M86 anti-α-Gal antibody (available from Vector Laboratories), which are both specific for the α-Gal epitope, or by Sambucus nigra lectin (SNA), which is specific for glycans with terminal sialic acids.

The invention is now described in greater detail by way of examples and with reference to the figures, which show in
- Fig. 1 micrographs (scale bar = 100 µm) of glycan-specific staining,
- Fig. 2 photos of dot blots to determine binding of pre-formed human serum antibodies to matrix material,
- Fig. 3 a graphical analysis of the results of Fig. 2., and in
- Fig. 4 micrographs (scale bar = 100 µm) showing human umbilical vein endothelial cells growing on decellularized (A) or decellularized and oxidized (B) porcine pulmonary heart valve leaflets

### Example: Producing an implant from porcine matrix material

As an example for a xenogeneic acellular matrix, a section of a decellularized porcine pulmonary artery was used. The original pulmonary artery was decellularized by SDS/SD (0.5 wt.-% each in aqueous solution) to generate the acellular matrix.

The acellular matrix was oxidized by treatment with 10 mM sodium periodate in sterile water (AMPUWA Sterilwasser, obtainable from Fresenius Kabi AG, Germany) for 3 h at 37°C under agitation in a rotary shaker at 150 rpm. As a result, the oxidized acellular matrix was obtained.

Subsequently, the oxidized acellular matrix was washed in 10 mM ethylene glycol for 1h to quench the oxidizing agent followed by three iterations for ten minutes each in phosphate-buffered saline (PBS) for removing the ethylene glycol and oxidizing agent and immediately subsequently was immersed in 1 mM aminooxy-biotin in PBS, pH 7.6, for at least 6 h at 37°C and 150rpm followed by three washing steps in PBS for ten minutes each to produce a matrix material according to the invention. Herein, the aminooxy-biotin represents the agent that is reactive with carbonyl groups, herein generally also termed saturating agent.

For detection of glycans, staining by Isolectin B4 and M86 anti-α-Gal antibody (available from Vector Laboratories), which are both specific for the α-Gal epitope, and by Sambucus nigra lectin (SNA), which is specific for terminal sialic acid glycans, were performed on samples of i) the acellular matrix derived from wild-type pigs (WT), of ii) the decellularized matrix derived from wild-type pigs which was treated by the process of the invention (WT biotinylated), and of iii) a section of pulmonary artery originating from a genetically manipulated GGTA1-Knock-out pig which was also decellularized (GGTA1-KO).

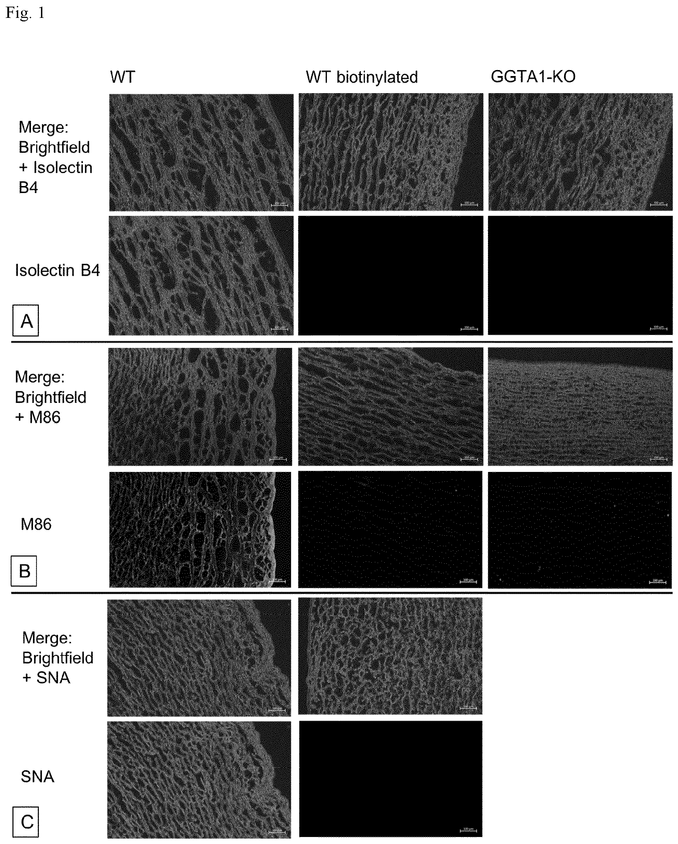

The results are depicted in the micrographs of Fig. 1 (A-C), showing that the acellular matrix without oxidation nor reaction with the saturating agent (WT) was strongly detected with Isolectin B4, the M86 anti-α-Gal antibody and SNA, showing that it contained both the glycans comprising α-Gal and terminal sialic acid, whereas only the implant produced by the process according to the invention (WT biotinylated) shows a significantly reduced or abolished detection by the lectins and the antibody. The non-biotinylated GGTA1-KO acellular matrix (GGTA1-KO) was not detected by Isolectin B4 or the M86 anti-α-Gal antibody, confirming absence of the α-Gal epitope.

For analyzing the presence of antigenicity of matrix materials, sections of differently treated matrix materials were mechanically minced and enzymatically solubilized, then bound by dotting onto a membrane in technical triplicates. The membrane-bound dots were then exposed to 5 different human sera to test for binding of serum-born antibodies to the matrix material.

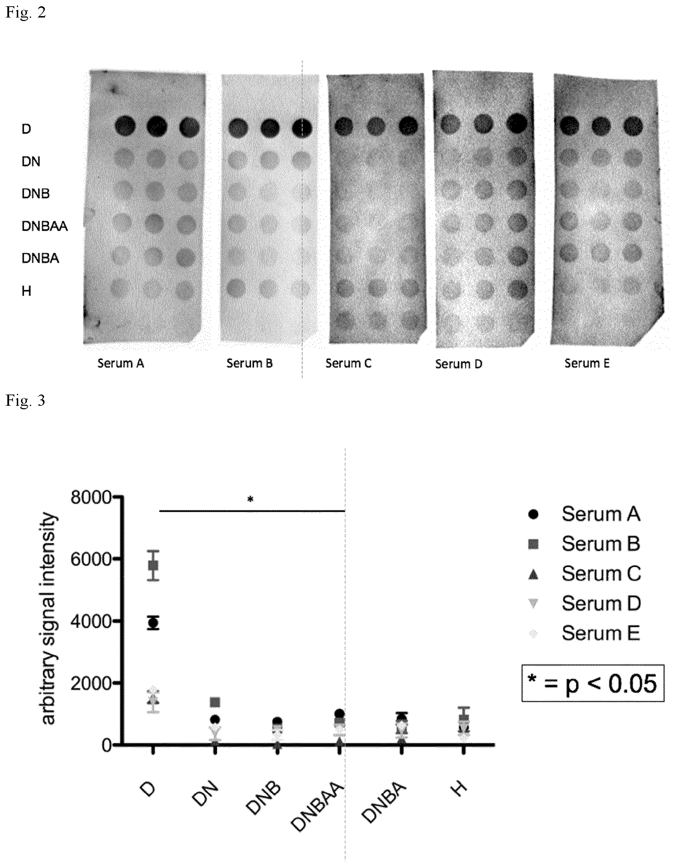

The following matrix materials were analyzed and are shown in Fig. 2: Sections of the same decellularized porcine pulmonary artery (D, comparative, acellular matrix material), sodium periodate-oxidized acellular matrix (DN, comparative), sodium periodate-oxidized acellular matrix reacted with amino-oxy biotin (B) as saturating agent directly subsequent to oxidation (DNB), sodium periodate-oxidized acellular matrix reacted with amino-oxy biotin as saturating agent in presence of anthranilic acid (AA) as catalyst directly subsequent to oxidation (DNBAA), sodium periodate-oxidized acellular matrix reacted with amino-oxy biotin as saturating agent in presence of aniline (A) as catalyst directly subsequent to oxidation (DNBA), decellularized human pulmonary artery (H, negative control).

The membranes were incubated in 5 different human sera (Serum A to Serum D), detection of bound serum antibodies was done by incubation with HRP-conjugated secondary anti-human IgG, IgM, IgA antibody. This semi-quantitative analysis was evaluated by measuring signal intensities.

Fig. 3 shows a graph of the signal intensities, confirming that for all sera binding to the acellular porcine matrix (D) was strongly higher than binding to the oxidized porcine matrix (DN) or to the oxidized and biotinylated porcine acellular matrices (DNB, DNBA, DNBAA). Detection of the oxidized porcine matrix (DN) or the oxidized and biotinylated porcine acellular matrices (DNB, DNBA, DNBAA) by the different sera were in the same range as detection of the human (H) matrix which served as a negative control.

This shows that the process of the invention produces an acellular matrix that effectively avoids the high antigenicity of porcine decellularized matrix material, at least in relation to pre-formed antibodies. This result also shows that these sera did not contain significant levels of antibodies that could bind to other antigenic sites than those that are minimized or abolished by the process of the invention.

In summary, immunological analyses of matrix materials produced by the process of the invention have shown that glycan epitopes were effectively inactivated, e.g. destroyed or masked.
As the process of the invention is not limited to reacting with specific glycans of the acellular matrix material, the process minimizes or effectively abolishes the antigenicity of glycans in general of this matrix material.

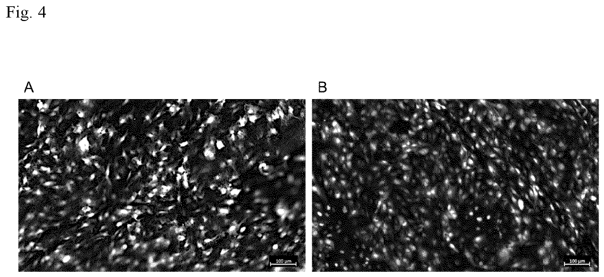

In Fig. 4 human umbilical vein endothelial cells (HUVECs) were seeded on acellular pulmonary heart valve leaflets obtained from WT pigs with (A) or without (B) treatment by the process according to the invention. The cells were cultivated for 6 days in total and show proliferation to a confluent monolayer in both cases. This suggests that the process of the invention avoids cross-linking of the matrix, which allows for the matrix to have a structure that can be colonized by cells of the human recipient, and, preferably, the matrix can after colonization with cells be re-modelled and grow within the human recipient.

## Claims

1. Process for producing an acellular implant from a matrix material of non-human animal origin comprising a step of decellularizing a matrix material to generate an acellular matrix, a step of oxidizing the acellular matrix by treatment of the acellular matrix with an oxidant to generate an oxidized acellular matrix, a step of reacting the oxidized acellular matrix with an agent that is reactive with carbonyl groups and is non-antigenic.

2. Process according to claim 1, **characterized in that** the step of oxidizing comprises contacting the acellular matrix with an aqueous solution of an oxidizing agent which specifically oxidizes the glycans moieties to aldehydes, preferably sodium periodate.

3. Process according to one of the preceding claims, **characterized in that** the step of oxidizing is in the presence of the agent that is reactive with carbonyl groups and is non-antigenic.

4. Process according to one of the preceding claims, **characterized in that** the agent that is reactive with carbonyl groups and is non-antigenic is a primary amine, an aminooxy-compound, or a hydrazide that is reactive to the carbonyl groups of glycans, or a mixture of at least two of these.

5. Implant consisting of an acellular matrix of non-human animal origin, obtainable by a process according to one of the preceding claims.

6. Implant according to claim 5, **characterized in that** an agent that is reactive with carbonyl groups and is non-antigenic is bound to the glycans of the implant and the glycans are inaccessible for the immune system of a human recipient.

7. Implant according to one of claims 5 to 6, **characterized in that** the agent that is reactive with carbonyl groups and is non-antigenic is biotin, a reactive biotin-derivative compound or an amino acid or a protein.

8. Implant according to one of claims 5 to 7, **characterized in that** the implant has no glycans that have antigenicity in human recipients.

9. Implant according to one of claims 5 to 8, **characterized in that** the proteinaceous extracellular matrix comprising the implant is devoid of artificial cross-linkages.
